# EUROPEAN PATENT SPECIFICATION

(11) **EP 1 910 262 B1**
(45) Date of publication and mention of the grant of the patent: **03.03.2010**
(21) Application number: 06765943.3
(22) Date of filing: 29.06.2006
(51) Int. Cl.: C07C 49/637, C11D 3/50

(54) **NAPHTHALENONE DERIVATIVE WITH POWDERY-IONONE TYPE ODOURS**
NAPHTHALINONDERIVAT MIT GERÜCHEN VOM PULVRIGEN IONON-TYP
DERIVE NAPHTALENONE PRESENTANT DES ODEURS DE TYPE IONONE PULVERULENTE

(30) Priority: 19.07.2005 WO PCT/IB2005/002062
(43) Date of publication of application: 16.04.2008
(73) Proprietor: FIRMENICH SA, CH-1211 Geneva 8 (CH)
(72) Inventor: FEHR, Charles, CH-1290 Versoix (CH); FARRIS, Iris, CH-1290 Versoix (CH)
(74) Representative: Carina, Riccardo Filippo
(86) International application number: PCT/IB2006/052177
(87) International publication number: WO 2007/010420

(56) References cited:
- HATZELLIS, KONSTANTINOS ET AL: "Correction of the Structure of a New Sesquiterpene from Cistus creticus ssp. creticus" JOURNAL OF NATURAL PRODUCTS , 67(12), 1996-2001 CODEN: JNPRDF; ISSN: 0163-3864, 2004, XP002409536
- FRANCK-NEUMANN, MICHEL ET AL: "Synthesis of mono- and polycyclic tricarbonyliron cyclohexa-2,4-dienone complexes and phenols from tosylhydrazones of acyclic dienone complexes" SYNLETT , (12), 2054-2058 CODEN: SYNLES; ISSN: 0936-5214, 2002, XP002409537
- KING, JOHN ET AL: "A new synthesis of 12-O-methylroyleanone" TETRAHEDRON LETTERS , 31(36), 5221-4 CODEN: TELEAY; ISSN: 0040-4039, 1990, XP002409538
- KHODABOCUS, AHMAD ET AL: "Substituent control of stereochemistry in the divinylketene- cyclohexadienone cyclization" JOURNAL OF THE CHEMICAL SOCIETY, CHEMICAL COMMUNICATIONS , (12), 783-4 CODEN: JCCCAT; ISSN: 0022-4936, 1989, XP002409539
- BANERJEE, AJOY K. ET AL: "A formal total synthesis of (.+-.)-herbertene" JOURNAL OF THE CHEMICAL SOCIETY, PERKIN TRANSACTIONS 1: ORGANIC AND BIO-ORGANIC CHEMISTRY (1972-1999) , (8), 2485-90 CODEN: JCPRB4; ISSN: 0300-922X, 1988, XP002409540
- OHLOFF, G. ET AL: "Zur STereochemie der Geruchswahrnehmung von 1-Dekalon-Derivaten un ihren oxaananalogen Verbindungen" HELVETICA CHIMICA ACTA, vol. 118, no. 4, 1976, pages 1140-1157, XP002409541 cited in the application

## Description

### Technical field

The present invention relates to the field of perfumery. More particularly, it concerns a compound of formula (I) as defined below. Furthermore, the present invention concerns the use of said compound in the perfumery industry as well as the compositions or articles containing said compound.

### Prior art

In document US 3072709, the inventor mentions that 2,5,5,8a-tetramethyl-3,5,6,7,8,8a-hexahydro-1(2H)-naphthalenone, i.e. a compound comprised in the general formula of the present invention, can be a useful perfuming ingredient (no odour description provided). However, a few years later, the inventor of US 3072709 published a paper (J.Org.Chem., 1971, 1195) stating that by applying the protocol described in the US patent in fact it was not possible to obtain 2,5,5,8a-tetramethyl-3,5,6,7,8,8a-hexahydro-1(2H)-naphthalenone. Therefore, US 3072709 mentioned an erroneous structure, and the product effectively disclosed was 3,4,4a,7,8,8a-hexahydro-2α,4aβ,5,8aβ-tetramethyl-1(2H)-naphthalenone, and not the invention's compound.

In conclusion, all the compounds of the present invention are new, except for 3,5,6,7,8,8α-hexahydro-5,5,8a-trimethyl-1(2H)-naphthalenone (see K.Hatzellis et al, J.Nat.Prod, 67, 1996, 2004 or J.King et al, Tet.Lett, 31, 5221, 1990), (8aS)-6,7,8,8a-tetrahydro-2,5,5,8a-tetramethyl-1(5H)-naphthalenone or its (8aS) isomer(see M.Frank-Neumann et al, Synlett, 2054, 2002, or A.Banerjee et al, J.Chem.Soc Perkin 1, 8, 2485, 1988 or A.Khodabocuset al, Chem.Comm, 783, 1989), all disclosed as chemical intermediates.

The compounds perhydro-2,5,5,8a-tetramethyl-1-naphthalenone and perhydro-5,5,8a-trimethyl-1-naphthalenone are the known perfuming ingredients having the closest structure to the invention's compounds (see Helv.Chim.Acta, **1976**, 1140). The first is described as having a strong amber odour, while the second is described as having a camphor odour. Said structural analogues have organoleptic properties that differ considerably from the one of the present invention, and therefore they cannot be considered as suggesting or anticipating the usefulness of the invention's compounds in the field of perfumery, and even less of any organoleptic properties of the present compounds.

### Description of the invention

We have now surprisingly discovered that a compound of formula 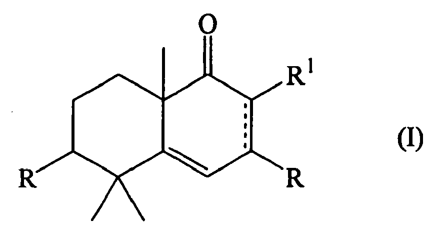
in the form of any one of its stereoisomers or a mixture thereof; and wherein the dotted line represents a single or double bond, R represents a hydrogen atom or methyl group and R¹ represents a hydrogen atom or a methyl or ethyl group.
can be used as perfuming ingredient, for instance to impart odour notes of the powdery, ionone and/or woody type, and therefore they are a first object of the invention.

According to a particular embodiment of the invention, the perfuming ingredient is a compound of formula 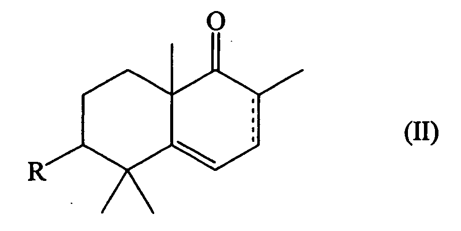 in the form of any one of its stereoisomers or a mixture thereof, and wherein R has the meaning indicated above, preferably a hydrogen atom.

Amongst the compounds of formula (II), wherein the dotted line represents a single bond, one may cite in particular the one having the methyl groups in the positions 2 and 8a in a relative configuration trans.

In particular, and as non-limiting examples, one may cite 2α,5,5,8aβ-tetramethyl-3,5,6,7,8,8a-hexahydro-1(2H)-naphthalenone which, to the contrary of perhydro-2,5,5,8a-tetramethyl-1-naphthalenone, does not have a strong amber odour, but rather an odour with woody (earthy-patchouli type), powdery and ionone (violet) notes. In fact, this unique combination of odour notes can give the impression of a nice woody, irone and slightly patchouli scent. This compound is particularly suitable to impart powdery, ionone notes and is capable of boosting the woody notes of ingredients or composition with which it is combined.

The two enantiomers of this compounds, namely (2S,8aR)-2,5,5,8a-tetramethyl-3,5,6,7,8,8A-hexahydro-1(2H)-naphthalenone and (2R,8aS)-2,5,5,8a-tetramethyl-3,5>6,7,8,8A-hexahydro-1(2H)-naphthalenone, although having organoleptic properties similar to one of the racemate, display different intensities, the (2S,8aR) enantiomer being more powerful than the (2R,8aS) enantiomer.

Another compound according to the invention is 2α,5,5,8a α-tetramethyl-3,5,6,7,8,8a-hexahydro-1(2H)-naphthalenone, which possesses also a powdery-ionone odour type, but distinguishes from the above compounds by having more pronounced bottom notes of the earthy type as well as being drier.

Furthermore, one may also cite 2,5,5,8a-tetramethyl-6,7,8,8a-tetrahydro-1(5H)-naphthalenone which possesses a ionone, rosy, damascone and earthy, woody odour, with bottom notes of the ionone, spicy (saffron) and earthy-rooty (vetiver) type. To the best of our knowledge, this compound is the only one having an odour combining at the same time a damascone and a woody character, and thus represents a unique and very advantageous tool for the perfumers palette in the creation of new scents.

Another example is 2α,6,5,5,8aβ-pentamethyl-3,5,6,7,8,8a-hexahydro-1(2H)-naphthalenone which possesses an odour with powdery, ionone and woody-cedar type odour notes.

Finally, one may also cite as invention's example 5,5,8a-trimethyl-3,5,6,7,8,8a-hexahydro-1(2H)-naphthalenone, which displays also an odour of the woody type slightly watery and floral.

Thus, the invention's compounds belong to the family of the powdery and/or woody ingredients and are particularly useful to the perfumers to impart woody, powdery, iononic and/or irone-like odours, reminding of wood in some cases, to the composition or article in which they are added.

According to a particular embodiment of the invention's the preferred compounds are 2,5,5,8a-tetramethyl-6,7,8,8a-tetrahydro-1(5H)-naphthalenone, (2S,8aR)-2,5,5,8a-tetramethyl-3,5,6,7,8,8A-hexahydro-1(2H)-naphthalenone or 2α,5,5,8aβ-tetramethyl-3,5,6,7,8,8a-hexahydro-1 (2H)-naphthalenone.

Another object of the present invention concerns the use of compound of formula (I) as perfuming ingredients. In other words it concerns a method to confer, enhance, improve or modify the odour properties of a perfuming composition or of a perfumed article, which method comprises adding to said composition or article an effective amount of at least a compound of formula (I). In particular the invention's compounds can be used as perfuming ingredient to confer, enhance, improve or modify the woody, powdery and/or ionone odour notes. By "use of a compound of formula (I)" it has to be understood here also the use of any composition containing compound (I) and which can be advantageously employed in perfumery industry as active ingredients.

Said compositions, which in fact can be advantageously employed as perfuming ingredient, are also an object of the present invention.

Therefore, another object of the present invention is a perfuming composition comprising:
i) as perfuming ingredient, at least one invention's compound as defined above;
ii) at least one ingredient selected from the group consisting of a perfumery carrier and a perfumery base; and
iii) optionally at least one perfumery adjuvant.

By "perfumery carrier" we mean here a material which is practically neutral from a perfumery point of view, i.e. that does not significantly alter the organoleptic properties of perfuming ingredients. Said carrier may be a liquid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting example solvents such as dipropyleneglycol, diethyl phthalate, isopropyl myristate, benzyl benzoate, 2-(2-ethoxyethoxy)-1-ethanol or ethyl citrate, which are the most commonly used.

Generally speaking, by "perfumery base" we mean here a composition comprising at least one perfuming co-ingredient.

Said perfuming co-ingredient is not of the formula (I). Moreover, by "perfuming co-ingredient" it is meant here a compound, which is used in perfuming preparation or composition to impart a hedonic effect. In other words such a co-ingredient, to be-considered as being a perfuming one, must be recognized by a person skilled in the art as being able to impart or modify in a positive or pleasant way the odour of a composition, and not just as having an odour.

The nature and type of the perfuming co-ingredients present in the base do not warrant a more detailed description here, which in any case would not be exhaustive, the skilled person being able to select them on the basis of its general knowledge and according to intended use or application and the desired organoleptic effect. In general terms, these perfuming co-ingredients belong to chemical classes as varied as alcohols, aldehydes, ketones, esters, ethers, acetates, nitriles, terpene hydrocarbons, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

For the compositions which comprise both a perfumery carrier and a perfumery base, other suitable perfumery carrier, than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company).

Generally speaking, by ``perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

An invention's composition consisting of at least one compound of formula (I) and at least one perfumery carrier represents a particular embodiment of the invention as well as a perfuming composition comprising at least one compound of formula (I), at least one perfumery carrier, at least one perfumery base, and optionally at least one perfumery adjuvant.

It is useful to mention here that the possibility to have, in the compositions mentioned above, more than one compound of formula (I) is important as it enables the perfumer to prepare accords, perfumes, possessing the odour tonality of various compounds of the invention, creating thus new tools for their work.

Preferably, any mixture resulting directly from a chemical synthesis, e.g. without an adequate purification, in which the compound of the invention would be involved as a starting, intermediate or end-product could not be considered as a perfuming composition according to the invention.

Furthermore, the invention's compound can also be advantageously used in all the fields of modern perfumery to positively impart or modify the odour of a consumer product into which said compound (I) is added. Consequently, a perfumed article comprising:
i) as perfuming ingredient, at least one compound of formula (I), as defined above; and
ii) a consumer product base ;
is also an object of the present invention.

For the sake of clarity, it has to be mentioned that, by "consumer product base" we mean here a consumer product which is compatible with perfuming ingredients. In other words, a perfumed article according to the invention comprises the functional formulation, as well as optionally additional benefit agents, corresponding to a consumer product, e.g. a detergent or an air freshener, and an olfactive effective amount of at least one invention's compound.

The nature and type of the constituents of the consumer product do not warrant a more detailed description here, which in any case would not be exhaustive, the skilled person being able to select them on the basis of its general knowledge and according to the nature and the desired effect of said product.

Examples of suitable consumer product bases include solid or liquid detergents and fabric softeners as well as all the other articles common in perfumery, namely perfumes, colognes or after-shave lotions, perfumed soaps, shower or bath salts, mousses, oils or gels, hygiene products or hair care products such as shampoos, body-care products, deodorants or antiperspirants, air fresheners and also cosmetic preparations. As detergents there are intended applications such as detergent compositions or cleaning products for washing up or for cleaning various surfaces, e.g. intended for textile, dish or hard-surface treatment, whether they are intended for domestic or industrial use. Other perfumed articles are fabric refreshers, ironing waters, papers, wipes or bleaches.

Some of the above-mentioned consumer product bases may represent an aggressive medium for the invention's compound, so that it may be necessary to protect the latter from premature decomposition, for example by encapsulation.

The proportions in which the compounds according to the invention can be incorporated into the various aforementioned articles or compositions vary within a wide range of values. These values are dependent on the nature of the article to be perfumed and on the desired organoleptic effect as well as the nature of the co-ingredients in a given base when the compounds according to the invention are mixed with perfuming co-ingredients, solvents or additives commonly used in the art.

For example, in the case of perfuming compositions, typical concentrations are in the order of 0.001 % to 25 % by weight, or even more, of the compounds of the invention based on the weight of the composition into which they are incorporated. Concentrations lower than these, such as in the order of 0.01% to 15% by weight, can be used when these compounds are incorporated into perfumed articles, percentage being relative to the weight of the article.

### Examples

The invention will now be described in further detail by way of the following examples, wherein the abbreviations have the usual meaning in the art, the temperatures are indicated in degrees centigrade (°C); the NMR spectral data were recorded in CDCl₃ (if not stated otherwise) with a 360 or 400 MHz machine for ¹H and ¹³C, the chemical displacements δ are indicated in ppm with respect to TMS as standard, the coupling constants J are expressed in Hz.

### Example 1

### Synthesis of compounds of formula (I)

### a) 2α,5,5,8aβ-tetramethyl-3,5,6,7,8,8α-hexalydro-1(2H)-naphthalenone

A solution of EtAlCl₂ in hexane (1M; 192 ml; 192 mmol) was added under N₂ in 12 min to a cooled (-10 to -5°C), stirred solution of 2-methyl-4-(2,6,6-trimethyl-1-cyclohexen-1-yl)butanal (40.0 g; 192 mmol) in CH₂Cl₂ (500 ml). The reaction mixture was stirred for 30 min at 0°C , and treated in 7 minutes with chloral (37.5 ml; 56.6 g; 384 mmol). The reaction mixture was stirred for 90 min at 0°C and poured under stirring into an Erlenmeyer flask containing 5% HCl, ice and Et₂O. After stirring for 5 min, the phases were separated, and the organic phase was washed successively with H₂O (2x), saturated aqueous NaHCO₃ and brine dried (Na₂SO₄) and evaporated (62.9 g). Bulb-to-bulb distillations (oven temp. 125°C/0.02 mb or) gave the crude product. Purification of the crude product by two flash chromatographies on SiO₂ (400 g), using cyclohexane/AcOEt 98:2 afforded 29.64 g of desired compound (75%).

| | |
|---|---|
| ¹H-NMR: | 1.05 (*d, J* = 6, 3H); 1.08 (s, 3H); 1.18 (s, 3H); 1.21 (*m,* 1H); 1.37 (*s*, 3H); 1.41-1.52 (*m*, 2H); 1.59 (*m*, 1H); 1.68-1.82 (*m*, 2H); 2.02 (*m*, 1H); 2.61 (*m*, 1H); 2.99 (*m,* 1H); 5.54 (*m*, 1H) |
| ¹³C-NMR: | 216.6 (*s*); 149.1 (*s*); 117.7 (*d*); 47.8 (*s*); 41.0 (*t*); 37.2 (*d*);. 36.5 (*s*); 35.8 (*t*); 33.4 (*t*); 32.3 (*q*); 29.6 (*q*); 27.6 (*q*); 18.0 (*t*); 14.4 (*q*)*.* |

### b) 2α,5,5,8aα-tetramethyl-3,5,6,7,8,8a-hexahydro-1(2H)-naphathalenone

A solution of the compound obtained under a) (4.20 g; 20.40 mmol) in THF (5 ml) was treated at -30 to -20°C in 10 min with a solution of LDA (1.1 equiv; prepared from BuLi (1.62 M in hexane; 21.3 mmol) and diisopropylamine (22,3 mmol) in THF (25 ml)).The solution was stirred at -10°C for 20 min, cooled at -78°C and transferred via dropping funnel drop-wise (in 10 min) into a cooled (-78°C) solution of 2-butanol (15.0 g; 203.8 mmol) in THF (50 ml) (20.64 g (24.1 ml)); 190.0 mmol). The clear solution was stirred at -10°C for 10 min and poured under vigorous stirring into a two-phase system 5% HC1/Et₂O. The phases were separated and the organic phase was washed with saturated aqueous NaHCO₃ solution brine solution (2x), dried (Na₂SO₄), evaporated (4.18 g) and bulb-to-bulb distilled (oven temp. 125°C/0.02 mbar). Yield: 4.05 g of the desired compound (yield: 96%).

| | |
|---|---|
| ¹H-NMR: | 1.09 *(d, J =* 6, 3H); 1.13 (*s*, 3H); 1.16 (*s*, 3H); 1.22-1.37 (*m*, 2H); 1.30 (*s*, 3H); 1.46 (*m*, 1H): 1.55 (*m*, 1H); 1.72-1.86 (*m*, 2H); 2.00 (*m*, 1H); 2.27-2.42 (*m*, 2H;); 5.87 (*m*, 1H) |
| ¹³C-NMR: | 218.5 (*s*); ,150.3 (*s*); 119.3 (*d*); 48.4 (*s*); 40.7 (*d*); 40.6 (*t*); 36.3 (*t*); 35.6 (*s*); 32.4 (*q*); 30.2 (*q*); 30.1(*t*); 23.7 (*q*); 18.2 (*t*); 14.9 (q). |

### c) 2,5,5,8a-tetramethyl-6,7,8,8a-tetrahydro-1(5H)-naphtfanlenone

### i) 1,2,3,4,4a,7-hexahydro-1,1,4a,6-tetramethyl-5-(trimethylsilyloxy)naphthalene

A solution of 2a,5,5,8aβ-tetramethyl-3,5,6,7,8,8a-hexahydro-1(2H)-naphthalenone (19.57 g; 95.02 mmol) in THF (120 ml) was treated at -30 to -20°C in 50 min with a solution of LDA (1.1 equiv; prepared from BuLi (1.44 M in hexane; 104.5 mmol) and diisopropylamine (11.52 g; 114.1 mmol) in THF (100 ml)).The yellow-orange solution was stirred at -10°C for 1 h, cooled at -30°C and treated in 5 min with TMSC1 (20.64 g; 190.0 mmol). The clear solution was stirred at -10°C for 10 min and poured under vigorous stirring into a two-phase system of saturated aqueous NaHCO₃ solution and pentane. The phases were separated and the organic phase was washed twice with saturated aqueous NaCl solution, dried (Na₂SO₄), evaporated (26.75 g) and bulb-to-bulb distilled (oven temp. 125°C/0.02 mbar). Yield: 25.41 g (96%).
¹H-NMR (characteristic signals): Me singlets at 1.11, 1.16, 1.24 and 1.58.

### ii) 2,5,5,8aβ-tetramethyl-2α-[trimethylsilyloxy]-3,5,6,7,8,8A-hexahydro-1(2H)-naphthalenone

A. solution of 1,2,3,4,4a,7-hexahydro-1,1,4a,6-tetramethyl-5-(trimethylsilyloxy) naphtalene (25.41g; 91.4 mmol) in CH₂Cl₂ (200ml) was treated drop-wise (90 min) at 0°C with a solution of 70% mCPBA (25.49 g; 103.4 mmol) in CH2Cl₂ (250 ml). The reaction was quenched with a 10% aqueous Na₂SO₃ solution (200 ml), the phases separated and the organic phase washed (2x 5% NaOH, then 2x saturated aqueous NaCl), dried (Na₂SO₄) and evaporated to give 26.47 g of the desired product.
¹H-NMR (characteristic signals): Me singlets at 0.09, 1.13, 1.16, 1.32 and 1.40.

### iii) 2,5,5,8aβ-tetramethyl-1-oxo-1,2,3,5,6,7,8,8A-octahydro-2α-naphthalenyl acetate

A solution of crude 2,5,5,8aβ-*tetramethyl*-2α-[trimethylsilyloxy]-3,5,6,7,8,8A-hexahydro-1(2H)-naphthalenone (26.47g; max. 91.4 mmol) in Ac₂O (100ml) was treated at 25°C with *p*TsOH.H₂O (1.0 g) and H₂O (2 ml) and stirred for 1 hour. More water was added (100 ml) and the reaction mixture stirred for 30 minutes The product was extracted with Et₂O and washed successively with 5% NaOH (3x), H₂O and saturated aqueous NaCl (2x), dried (Na₂SO₄) and evaporated (22.73 g) and bulb-to-bulb distilled (oven temp. 125°C/0.02 mbar) to give 21.98 g (yield: 80%) of the desired product.
¹H-NMR (characteristic signals): Me singlets at 1.13, 1.17, 1.38 and 1.59.

### iv) 2,5,5,8a-tetramethyl-6,7,8,8a-tetrahydro-1(5H)-naphthalenone

A solution of 2,5,5,8aβ-*tetramethyl-*1-oxo-1,2,3,5,6,7,8,8A-octahydro-2α-naphthalenyl acetate (3.37 mmol) in DBU (1.03 g; 6.74 mmol)was heated at 120°C for 6 hours. The reaction mixture was cooled at room temperature and poured into 5% HCl, ice and Et₂O. The product was extracted with Et₂O and washed successively with H₂O saturated aqueous NaHCO₃ solution (2x), and saturated aqueous NaCl, dried (Na₂SO₄) and evaporated (0.79 g). Purification by flash chromatography on SiO₂ (50 g), using cyclohexane/AcOEt 96:4 afforded 473 mg of the desired product (69%) which could be crystallized from heptane.

| | |
|---|---|
| ¹H-NMR: | 1.16 (*s*, 3H); 1.23 (*s*, 3H); 1.28 (*m*, 1H); 1.33 (*s*, 3H); 1.37(*m*, 1H); 1.53 (*m*, 1H); 1.68-1.64 (*m,* 1H); 1.85(*m*, 1H); 1.86 (*s*, 1H); 2.14 (*m*, 1H); 6.06 (*d, J* = 6, 1H); 6.78 *(d, J* = 6, 1H). |
| ¹³C-NMR: | 208.5 (*s*); 163.0(*s*); 138.0 (*d*); 129.5 (*s*); 115.0 (*d*); 50.9 (*s*); 40.7 (*t*); 36.8 (s); 33.6 (*t*); 31.8 (*q*); 29.6 (*q*); 27.8 (*q*); 18.1 *(t);* 15.4 *(q).* MS: 204 (M⁺; 100), 189 (74), 161 (53), 148(42), 135(70), 119(19), 105(28), 91(37). |

### d) (2R,8aS)-2,5,5,8a-tetramethyl-3,5,6,7,8,8a-hexahydro-1(2H)-naphthalenone

### i) (2R)-2-methyl-4-(2,6,6-trimethyl-1-cyclohexen-1-yl)butanol

A solution of acid (2R)-2-methyl-4-(2,6,6-trimethyl-1-cyclohexen-1-yl)butanoic acid (obtained by optical resolution of the racemic acid with (-)-(S)-1-phenylethylamine) (3.99 g; 17.82 mmol) in Et₂O (15 ml) was added dropwise under N₂ to a stirred suspension of LiAlH₄ (1.36 g; 35.64 mmol) in Et₂O (35 ml). The gently refluxing reaction mixture was maintained at reflux for 2 hours, cooled at 0°C and treated carefully with 1.5 ml of water, 1.5 ml of 5% NaOH and 3x 1.5 ml of water. The suspension was dried (Na₂SO₄) filtered (Celite), concentrated (3.73 g) and the product was bulb-to-bulb distilled (oven temp. 130°C/0.04 mbar) affording 3.51 g of (2R)-2-methyl-4-(2,6,6-trimethyl-1-cyclohexen-1-yl)butanol (purity 97%; yield 91%).[α]_{D}²⁰ (CHCl₃; c = 1.57) +8.9 (97% ee).

### ii) (2R)-2-methyl-4-(2,6,6-trimethyl-1-cyclohexen-1-yl)butanal

A solution of DMSO (2.65 ml; 2.91 g; 37.24 mmol) in CH₂Cl₂ (15 ml) was added at -78°C in 15 min to a solution of oxalyl chloride (2.23 ml; 3.29 g; 25.90 mmol) in CH₂Cl₂ (40 ml). After 20 min a solution of (2R)-2-methyl-4-(2,6,6-trimethyl-1-cyclohexen-1-yl)butanol (3.50 g; 97% pure; 16.19 mmol) in CH₂Cl₂ (40 ml) was added dropwise to the aforementioned mixture in such a way that the temperature never exceeds -65°C. After introduction (30 min), the milky suspension was stirred for 30 minutes at-78°C, then NEt₃ (10.84 ml; 7.86 g; 77.71 mmol) was added dropwise and the temperature was allowed to reach 0°C. The mixture was poured into water and the product extracted with pentane, washed with brine (2x), dried (Na₂SO₄ and evaporated. The product was bulb-to-bulb distilled (oven temp. 100°C/0.6 mbar): 3.16 g of (2R)-2-methyl-4-(2,6,6-trimethyl-1-cyclohexen-1-yl)butanal (purity 96%; yield 90%). [α])D²⁰ (CHCl₃; c = 1.10) -21.8.

### iii) (2R,8aS)-2,5,5,8a-tetramethyl-3,5,6,7,8,8a-hexahydro-1 (2H)-naphthalenone

Proceeding as described in example 1a, (2R)-2-methyl-4-(2,6,6-trimethyl-1-cyclohexen-1-yl)butanal (1.61 g; 7.76 mmol) was converted with EtAlCl₂ in hexane (1M; 7.76 ml; 7.76 mmol) into the corresponding naphthalenol (1.16 g; 72% after chromatography; [α]D²⁰ (CHCl₃; c = 1.52) -119; 97% ee by GC), which was oxidized (*Jones* oxidation) (903 mg; 4.34 mmol), to afford the desired compound ([α]_{D}²⁰ (CHCl₃; c = 2.50) -130 (97% ee)) (877 mg; yield=98%).
The NMR spectra were the same as for the racemic compound (Example 1 a).

### e) (2S,8aR)=2,5,5,8a-tetramethyl-3,5,6,7,8,8a-hexahydro-1(2H)-naphthalenone

It was proceeded as in Example 1d, but starting from acid (2S)-2-methyl-4-(2,6,6-trimethyl-1-cyclohexen-1-yl)butanoic acid (obtained by optical resolution of the racemic acid with (-)-(*S*)-1-phenylethylamine).
The NMR spectra were the same as for the racemic compound (Example la).

### Example 2

### Synthesis of 2a,6,5,5,8aβ-pentamethyl-3,5,6,7,8,8a-hexahydro-1 (2H)-naphthalenone

### Preparation of 2-methyl-2-12-(2,5,6, 6-tetramethyl-1-cyclohexen-1-yl)ethyl]lorirane

Dimethyl sulfate (31:52 g; 250.2 mmol) was added under N₂ in 10 minutes to a slowly stirred solution of Me₂S (16.80 g; 271.0 mmol) in DMSO (200 ml). The temperature rose to 46°C (after 30 minutes) and was let attain room temperature. Under vigourous stirring, NaOH grains (60 g; 1.50 mol) were added in one portion, followed by the rapid addition of (±)-dihydro-β-irone (43.36. g; 208.5 mmol). Stirring was continued for 67 hours (97% conversion).
The reaction mixture was poured into ice/saturated aqueous NaCl and the product extracted with pentane (2x) and washed with water (4x). The organic phases were stirred with 10% ammonia for 2 hours (reaction with excess dimethyl sulfate), separated and washed with saturated aqueous NaCl (4x), dried (Na₂SO₄ and evaporated. The crude product was bulb-to-bulb distilled (oven temp. 100-120°C/0.01 mbar), giving the corresponding epoxide (38.04 g 82% yield) as a mixture of diastereomers (ca. 1:1; not GC-separated).
The 95% pure product was pure enough for the next step, but it was also re-purified by chromatography (SiO₂), using cyclohexane/AcOEt = 98:2, followed by bubbling N₂ through the pure compound.

| | |
|---|---|
| ¹³C-NMR: | 136,4 (*s*); 127.1 (2*s*); 57.1 (*s*); 53.8 (2*t*); 39.3 (*d*); 38.2 (*s*); 37.2 *(*2*t);* 31.6 (2*t);* 27.2 (2*t*); 26.9 (2*q*); 24.2 (*t*); 21.7 (2*q*); 20.9 (*q*); 19.8 (*q*); 16.6 (*q*). |

### Preparation of 2-methyl-4-(2.5.6.6-tetrametyl-1-cyclohexen-1-vl)butanal

A mixture of Filtrol G 13 (2.60 g) and epoxide obtained in step 1 (26.48 g; 119.0 mmol) in toluene (260 ml) was heated at 35°C for 30 minutes, then cooled to room temperature and filtered over Celite, evaporated (27.04 g) and bulb-to-bulb distilled (oven temp. 125°C/0.1 mbar) , giving the corresponding aldehyde (17.09 g; 80% pure by GC) as a mixture of diastereomers (ca. 1:1). The product was purified by chromatography (SiO₂ (320 g)), using cyclohexane/AcOEt = 95:5. Pure 8: 13.23 g (50% yield).

| | |
|---|---|
| ¹³C-NMR: | 205.1 (*d*); 136.8 (*s*); 127.2 (2*s*); 47.2 (*d*); 39.3 (*d*); 38.1 (s); 31.6 (*t*); 31.1 (*t*); 27.2 (*t*); 27.0 (*q*); 26.4 (*t*); 21.8 (*q*); 20.0 (2*q*); 16.6 (*q*); 13.2 (2*q*). |

### Preparation of the title compounds

A solution of EtAlCl₂ in hexane (1 M; 22.52 ml; 22.52 mmol) was added under N₂ in 10 min to a cooled (-15° C to -5° C), stirred solution of the aldehyde obtained in step 2 (5.0 g; 22.52 mmol) in CH₂Cl₂ (100 ml). After stirring for 30 minutes at -5° C, the reaction mixture was poured under stirring into an Erlenmeyer flask containing 5% HCl, ice and Et₂O. The phases were separated, and the organic phase was washed successively with H₂O, saturated aqueous NaHCO₃ and saturated aqueous NaCl, dried (Na₂SO₄ and evaporated (5.01 g; 2 diastereomers of the naphthalenol obtained). Without purification, the naphthalenol obtained was dissolved in acetone (70 ml), cooled at 0° C and treated drop-wise with Jones reagent (2.5 M; 9.91 ml; 1.1 equiv). After stirring the green suspension for 1 hour, the reaction mixture was poured under stirring into an Erlenmeyer flask containing saturated aqueous NaHCO₃ and pentane. The phases were separated and re-extracted 3 times with pentane, and the collected organic phases were washed successively with saturated aqueous NaHCO₃ 5% NaOH and saturated aqueous NaCl, dried (Na₂SO₄ and evaporated. The product was purified by three chromatography (SiO₂ (250 g)), using cyclohexane/AcOEt = 96:4, to yield the title naphthalenone in 70% yield.

| | |
|---|---|
| ¹³C-NMR: | 216.8 (*s*); 216.7 (*s*); 150.2 (*s*); 147.0 (*s*); 119.4 (*d*); 117.5 (*d*); 48.0 (*s*); 47.5 (s); 41.4 (*d*); 39.8 (*s*); 39.7 (*s*); 39.3 (*d*); 37.2 (2*d*); 35.7 (2*t*); 33.1 *(t).* 31.3 *(q);* 29.1 (*q)*; 28.5 (2*q*); 27.2 (*q*); 27.0 (2*t*); 25.0 (*t*); 23.7 (*q*); 16.9 (*q*); 15.6 (*q*); 14.4 (*q*); 14.2 (*q*). |

### Example 3

### Synthesis of 5,5,8a-trimethyl-3,5,6,7,8,8a-hexahydro-1(2H)-naphthalenone

### i) 5,5,8aβ-trimethyl-1-1,2,3,5,6,7,8,8a-octahydro-1α-naphthalenol

A solution of EtAlCl₂ in hexane (1 M; 8.56 ml; 8.56 mmol) was added under N₂ to a cooled (-10°C), stirred solution of 4-(2,6,6-trimethyl-1-cyclohexen-1-yl)butanal (1.66 g; 8.56 mmol) (M. P. Zink et al., Helv. Chim. Acta 1976, 59, 32) in CH₂Cl₂ (30 ml). The reaction mixture was stirred for 1 h at -8°C and was poured under stirring into 5% HCl, ice and Et₂O. The phases were separated, and the organic phase was washed successively with H₂O (twice), saturated aqueous NaHCO₃ and brine, dried (Na₂SO₄) and evaporated (1.64 g). Flash chromatography on SiO₂ (100 g), using cyclohexane/AcOEt 95:5 afforded 720 mg of naphthalenol (purity 94%; yield 41 %).

| | |
|---|---|
| ¹³C-NMR: | 147.2 (s); 118.0 (d); 75.6 (d); 41.1 (t); 39.2 (s); 35.5 (s); 33.9 (t); 31.6 (q); 30.7 (q); 28.3 (q); 24.1 (t); 20.7 (t). 18.1 (t). |

### ii) 5,5,8a-trimethyl-3,5,6,7,8,8a-hexahydro-1(2H)-naphthalenone

The naphthalenol obtained above (303 mg; 94% pure; 1.47 mmol) was dissolved in acetone (5 ml), cooled at 0°C and treated drop-wise with Jones reagent (2.5 M; 0.70 ml; 1.76 mmol). After stirring the green suspension for 15 minutes at 0°, the reaction mixture was poured into 5% NaOH and extracted with pentane. The phases were separated and re-extracted twice with pentane and washed with water and brine, dried (Na₂SO₄) and evaporated (310 mg). The product was bulb-to-bulb distilled (oven temp. 90°C/0.5 mbar) to provide the desired compound (purity 95%; yield 86%).

| | |
|---|---|
| ¹³C-NMR: | 216.1 (s); 149.3 (s); 118.8 (d); 47.8 (s); 40.7 (t); 36.1 (s); 35.2 (t); 33.8 (t); 32.3 (q); 30.2 (q); 26.2 (q); 20.8 (t). 17.9 (t). |

### Example 4

### Preparation of a perfuming composition

A perfuming composition of the musky-floral type, for a detergent, was prepared by admixing the following ingredients :

| Ingredient | Parts by weight |
|---|---|
| Benzyl acetate | 60 |
| 10%* Carbinol acetate | 20 |
| 10%* Acetophenone | 5 |
| Aldehyde C 10 | 20 |
| Aldehyde C 12 | 60 |
| Aldehyde MNA | 80 |
| 10%* Methyl anthranilate | 20 |
| Cashmeran^{® 1)} | 100 |
| 10%* Cetalox^{® 2)} | 35 |
| Lemon oil | 40 |
| Citronellol | 200 |
| Verdyl propionate ³⁾ | 230 |
| Dihydromyrcenol | 800 |
| Geraniol essential oil | 40 |
| Lilial^{® 4)} | 200 |
| Neobutenone^{® 5)} | 5 |
| Rose oxide | 10 |
| 10%* Romascone^{® 6)} | 25 |
| Hexyl salicylate | 600 |
| 10%* 4-Methyl-3-decen-5-ol | 50 |
| Verdox^{® 7)} | 500 |
| | 3̅1̅0̅0̅ |

| | |
|---|---|
| * in dipropyleneglycol 1) 1,2,3,5,6,7-hexahydro-1,1,2,3,3-pentamethyl-4-indenone; origin: International Flavors & Fragrances, USA; 2) dodecahydro-3a,6,6,9a-tetramethyl-naphtho[2,1-b]furan; origin: Firmenich SA, Switzerland; 3) origin: Givaudan-Roure SA, Vernier, Switzerland. 4) 3-(4-tert-butylphenyl)-2-methylpropanal; origin: Firmenich SA, Switzerland; 5) 1-(5,5-dimethyl-1-cyclohexen-1-yl)-4-penten-1-one; origin: Firmenich SA, Switzerland; 6) methyl 2,2-dimethyl-6-methylene-1-cyclohexanecarboxylate; origin: Firmenich SA, Switzerland; 7) 2-tert-butyl-1-cyclohexyl acetate; origin: International Flavors & Fragrances, USA; | |

The addition of 400 parts by weight of 2α,5,5,8aβ-tetramethyl-3,5,6,7,8,8a-hexahydro-1 (2H)-naphthalenone to the above-described perfuming composition imparted to the latter a strong powdery-irone connotation, providing thus a scent with a remarkably increased volume and sweetness.

The addition of 400 parts by weight of 2,5,5,8a-tetramethyl-6,7,8,8a-tetrahydro-1(5H)-naphthalenone to the above perfuming composition resulted in a scent which had a clear damascone aspect, despite the fact that no damascone were presents. Furthermore, the flowery and the woody notes were also clearly more pronounced.

### Example 5

### Preparation of a perfuming composition

An eau-de-toilette for man was prepared by admixing the following ingredients :

| Ingredient | Parts by weight |
|---|---|
| 1-(1,2,3,4,5,6;7,8/3a-Octahydro-3,8-dimethyl-5-azulenyl)-1-methylethyl acetate | 50 |
| Linalyl acetate | 380 |
| Styrallyl acetate | 50 |
| Isobornyl acetate | 80 |
| Amylcinnamique aldehyde | 70 |
| 1%* Aldéhyde MNA | 10 |
| Armoise | 15 |
| Bay essential oil | 15 |
| Cetalox^{® 1)} | 20 |
| 1%* Raspberry ketone | 20 |
| Citral | 20 |
| Citron Sfuma | 260 |
| Citronellol | 100 |
| Cypres essential oil | 30 |
| Dihydromyrcenol | 200 |
| Eugenol | 25 |
| Cinnamon tree leaves oil | 30 |
| 10%* Linalyl formiat | 70 |
| 10%* Galbanum essential oil | 20 |
| Geraniol | 240 |
| Geranium oil | 50 |
| Habanolide^{® 2)} | 200 |
| Hédione^{® 3)} | 1000 |
| Iralia^{® 4)} Total | 120 |
| 10%* Isobutylquinoleine | 25 |
| Lavandin Grosso | 90 |
| Lilial^{® 5)} | 55 |
| Linalol | 420 |
| Lyral^{® 6)} | 50 |
| Mandarine oil | 270 |
| 10%* Methyl methylanthranilate | 40 |
| Mousse Cristal | 30 |
| Muscade oil | 60 |
| Nirvanol^{® 7)} | 60 |
| Patchouli oil | 380 |
| Orange essential oil | 350 |
| Romarin oil | 15 |
| Terpineol | 20 |
| 10%* Gamma undecalactone | 10 |
| Vanilline | 35 |
| Verdox^{® 8)} | 10 |
| 2,4-Dimethyl-3-cyclohexene-1-carbaldehyde | 5 |
| | 5̅0̅0̅0̅ |

| | |
|---|---|
| * in dipropyleneglycol 1) dodecahydro-3a,6,6,9a-tetramethyl-naphtho[2,1-b]furan; origin: Firmenich SA, Switzerland; 2) pentadecenolide; origin: Firmenich SA, Switzerland; 3) methyl dihydrojasmonate; origin: Firmenich SA, Switzerland; 4) mixture of methylionones isomers; origin: Firmenich SA, Switzerland; 5) 3-(4-tert-butylphenyl)-2-methylpropanal; origin: Firmenich SA, Switzerland; 6) 4/3-(4-hydroxy-4-methylpentyl)-3-cyclohexene-1-carbaldehyde; origin: International Flavors & Fragrances, USA; 7) 3,3-dimethyl-5-(2;2,3-trimethyl-3-cyclopenten-1-yl)-4-penten-2-ol; origin: Firmenich SA, Switzerland; 8) 2-tert-butyl-1-cyclohexyl acetate; origin: International Flavors & Fragrances, USA; | |

The addition of 400 parts by weight of 2α,5,5,8aβ-tetramethyl-3,5,6,7,8,8a-hexahydro-1(2H)-naphthalenone to the above-described eau de toilette exalted the woody character of the latter. This effect was even more perceivable upon evaporation during which the odour became more and more woody-powdery.
The addition of 400 parts by weight of 2,5,5,8a-tetramethyl-6,7,8,8a-tetrahydro-1(5H)-naphthalenone imparted to the eau de toilette a more pronounced woody but of fiffrent nature, more ethereal and floral. The new scent thus obtained had also a remarkable aerial aspect.

## Claims

1. A compound of formula 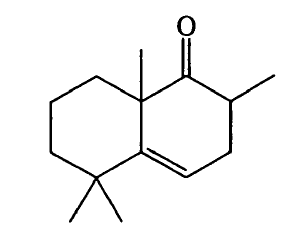 in the form of any one of its stereoisomers or a mixture thereof.

2. As a compound according to claim 1, 2α,5,5,8aβ-tetramethyl-3,5,6,7,8,8a-hexahydro-1(2H)-naphthalenone, (2S,8aR)-2,5,5,8a-tetramethyl-3,5,6,7,8,8A-hexahydro-1 (2H)-naphthalenone, (2R,8aS)-2,5,5,8a-tetramethyl-3,5,6,7,8,8A-hexahydro-1 (2H)-naphthalenone, 2a,5,5,8aa-tetramethyl-3,5,6,7,8,8a-hexahydro-1(2H)-naphthalenone.

3. A perfuming composition comprising:
i) as perfuming ingredient, at least a compound of formula (I): 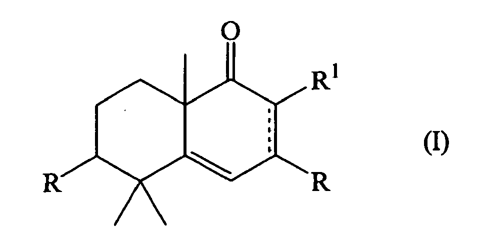 in the form of any one of its stereoisomers or a mixture thereof, and wherein the dotted line represents a single or double bond, R represents a hydrogen atom or methyl group and R¹ represents a hydrogen atom or a methyl or ethyl group;
ii) at least one ingredient selected from the group consisting of a perfumery carrier and a perfumery base; and
iii) optionally at least one perfumery adjuvant.

4. A perfuming composition according to claim 3, **characterised in that** said compound (I) is of formula 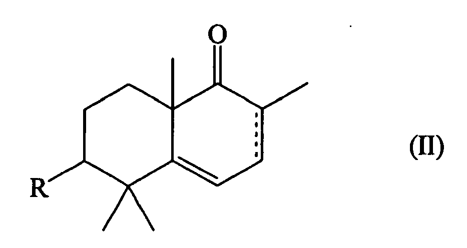 in the form of any one of its stereoisomers or a mixture thereof, wherein R and the dotted line have the meaning indicated in claim 3.

5. A perfuming composition according to claim 3, **characterised in that** said compound (I) is 2α,5,5,8aβ-tetramethyl-3,5,6,7,8,8a-hexahydro-1(2H)-naphthalenone, (2S,8aR)-2,5,5,8a-tetramethyl-3,5,6,7,8,8A-hexahydro-1(2H)-naphthalenone, (2R,8aS)-2,5,5,8a-tetramethyl-3,5,6,7,8,8A-hexahydro- (2H)-naphthalenone, 2α,5,5,8a α-tetramethyl-3,5,6,7,8,8a-hexahydro-1(2H)-naphthalenone, 2,5,5,8a-tetramethyl-6,7,8,8a-tetrahydro-1(5H)-naphthalenone, 2α,6,5,5,8aβ-pentamethyl-3,5,6,7,8,8a-hexahydro-1(2H)-naphthalenone or 5,5,8a-trimethyl-3,5,6,7,8,8a-hexahydro-1(2H)-naphthalenone.

6. A perfumed article comprising:
i) as perfuming ingredient, at least one compound of formula (I), as defined in claim 3; and
ii) a consumer product base.

7. A perfumed article according to claim 6, **characterised in that** the consumer product base is a solid or liquid detergent, a fabric softener, a perfume, a cologne or after-shave lotion, a perfumed soap, a shower or bath salt, mousse, oil or gel, a hygiene product, a hair care product, a shampoo, a body-care product, a deodorant or antiperspirant, an air freshener, a cosmetic preparation, a fabric refresher, an ironing water, a paper, a wipe or a bleach.

8. Use as perfuming ingredient of a compound of formula (I), as defined in claim 3.

## Patentansprüche

1. Verbindung der Formel 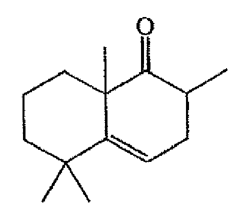 in der Form von irgendeinem ihrer Stereoisomere oder einem Gemisch davon.

2. Als Verbindung gemäß Anspruch 1 2α,5,5,8aβ-Tetramethyl-3,5,6,7,8,8a-hexahydro-1(2H)-naphthalinon, (2S,8aR)-2,5,5,8a-Tetramethyl-3,5,6,7,8,8a-hexahydro-1(2H)-naphthalinon, (2R,8aS)-2,5,5,8a-Tetramethyl-3,5,6,7,8,8a-hexahydro-1(2H)-naphthalinon, 2a,5,5,8aα-Tetramethyl-3,5,6,7,8,8a-hexahydro-1(2H)-naphthalinon.

3. Parfümierende Zusammensetzung, umfassend:
i) als parfümierenden Bestandteil mindestens eine Verbindung der Formel (I): 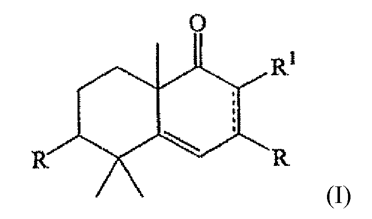 in der Form von irgendeinem ihrer Stereoisomere oder einem Gemisch davon, und wobei die gestrichelte Linie eine Einfach- oder Doppelbindung darstellt, R ein Wasserstoffatom oder eine Methylgruppe darstellt und R¹ ein Wasserstoffatom oder eine Methyl- oder Ethylgruppe darstellt;
ii) mindestens einen Bestandteil, ausgewählt aus der Gruppe, bestehend aus einem Träger für Parfümerie und einer Basis für Parfümerie, und
iii) wahlweise mindestens einen Hilfsstoff für Parfümerie.

4. Parfümierende Zusammensetzung gemäß Anspruch 3, **dadurch gekennzeichnet, daß** die Verbindung (I) die Formel 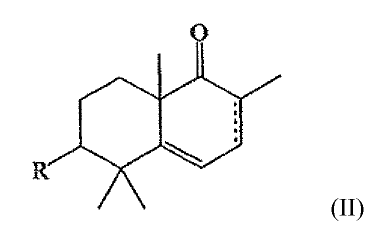 in der Form von irgendeinem ihrer Stereoisomere oder einem Gemisch davon hat, wobei R und die gestrichelte Linie die in Anspruch 3 angezeigte Bedeutung haben.

5. Parfümierende Zusammensetzung gemäß Anspruch 3, **dadurch gekennzeichnet, daß** die Verbindung (I) 2α,5,5,8aβ-Tetramethyl-3,5,6,7,8,8a-hexahydro-1(2H)-naphthalinon, (2S,8aR)-2,5,5,8a-Tetramethyl-3,5,6,7,8,8a-hexahydro-1(2H)-naphthalinon, (2R,8aS)-2,5,5,8a-Tetramethyl-3,5,6,7,8,8a-hexahydro-1(2H)-naphthalinon, 2α,5,5,8aα-Tetramethyl-3,5,6,7,8,8a-hexahydro-1(2H)-naphthalinon, 2,5,5,8a-Tetramethyl-6,7,8,8a-tetrahydro-1(5H)-naphthalinon, 2α,6,5,5,8aβ-Pentamethyl-3,5,6,7,8,8a-hexahydro-1(2H)-naphthalinon oder 5,5,8a-Trimethyl-3,5,6,7,8,8a-hexahydro-1(2H)-naphthalinon ist.

6. Parfümierter Gegenstand, umfassend:
i) als parfümierenden Bestandteil mindestens eine Verbindung der Formel (I), wie definiert in Anspruch 3; und
ii) eine Verbraucherproduktbasis.

7. Parfümierter Gegenstand gemäß Anspruch 6, **dadurch gekennzeichnet, daß** die Verbraucherproduktbasis ein festes oder flüssiges Detergens, ein Weichspüler, ein Parfüm, ein Kölnischwasser oder eine Aftershavelotion, eine parfümierte Seife, ein Dusch- oder Badesalz, -schaum, -öl oder -gel, ein Hygieneprodukt, ein Haarpflegeprodukt, ein Shampoo, ein Körperpflegeprodukt, ein Deodorant oder Antiperspirant, ein Luftverbesserer, eine kosmetische Zubereitung, ein Gewebeauffrischer, ein Bügelwasser, ein Papier, ein Wischtuch oder ein Bleichmittel ist.

8. Verwendung als parfümierender Gegenstand einer Verbindung der Formel (I), wie definiert in Anspruch 3.

## Revendications

1. Composé de formule 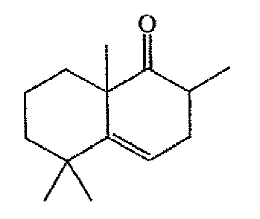 sous forme de n'importe lequel de ses stéréoisomères ou d'un de leurs mélanges.

2. Composé selon la revendication 1, qui est la 2α,5,5,8aβ-tétraméthyl-3,5,6,7,8,8a-hexahydro-1(2H)-naphtalénone, la (2S,8aR)-2,5,5,8a-tétraméthyl-3,5,6,7,8,8a-hexahydro-1(2H)-naphtalénone, la (2R,8aS)-2,5,5,8a-tétraméthyl-3,5,6,7,8,8a-hexahydro-1(2H)-naphtalénone, la 2α,5,5,8aα-tétraméthyl-3,5,6,7,8,8a-hexahydro-1(2H)-naphtalénone.

3. Composition parfumante comprenant:
i) en tant qu'ingrédient parfumant, au moins un composé de formule (I): 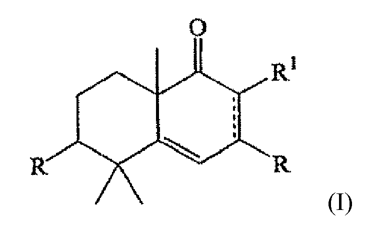 sous forme de n'importe lequel de ses stéréoisomères ou d'un de leurs mélanges, et où le trait en pointillé représente une liaison simple ou double, R représente un atome d'hydrogène ou un groupe méthyle et R¹ représente un atome d'hydrogène ou un groupe méthyle ou éthyle;
ii) au moins un ingrédient choisi dans le groupe constitué d'un véhicule de parfumerie et d'une base de parfumerie; et
iii) optionnellement, au moins un adjuvant d'usage courant en parfumerie.

4. Composition parfumante selon la revendication 3, **caractérisée en ce que** ledit composé (I) a pour formule 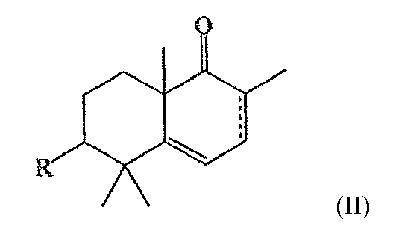 sous forme de n'importe lequel de ses stéréoisomères ou d'un de leurs mélanges, où R et le trait en pointillé ont la signification donnée dans la revendication 3.

5. Composition parfumante selon la revendication 3, **caractérisée en ce que** ledit composé (I) est la 2α,5,5,8aβ-tétraméthyl-3,5,6,7,8,8a-hexahydro-1(2H)-naphtalénone, la (2S,8aR)-2,5,5,8a-tétraméthyl-3,5,6,7,8,8a-hexahydro-1(2H)-naphtalénone, la (2R,8aS)-2,5,5,8a-tétraméthyl-3,5,6,7,8,8a-hexahydro-1(2H)-naphtalénone, la 2α,5,5,8aα-tétraméthyl-3,5,6,7,8,8a-hexahydro-1(2H)-naphtalénone, la 2,5,5,8a-tétraméthyl-6,7,8,8a-tétrahydro-l(5H)-naphtalénone, la 2α,6,5,5,8aβ-pentaméthyl-3,5,6,7,8,8a-hexahydro-1(2H)-naphtalénone ou la 5,5,8a-triméthyl-3,5,6,7,8,8a-hexahydro-1(2H)-naphtalénone.

6. Article parfumé comprenant:
i) en tant qu'ingrédient parfumant, au moins un composé de formule (I) selon la revendication 3; et
ii) une base de produit de consommation.

7. Article parfumé selon la revendication 6, **caractérisé en ce que** la base de produit de consommation est un détergent solide ou liquide, un adoucissant pour textile, un parfum, une eau de Cologne ou une lotion après-rasage, un savon parfumé, un sel, une mousse, une huile ou un gel de bain ou de douche, un produit d'hygiène, un produit de soin capillaire, un shampooing, un produit de soin corporel, un déodorant ou un anti-transpirant, un désodorisant d'air ambiant, une préparation cosmétique, une eau de linge, une eau de repassage, un papier, une lingette ou un agent de blanchiment.

8. Utilisation, en tant qu'ingrédient parfumant, d'un composé de formule (I) selon la revendication 3.
